# EUROPEAN PATENT SPECIFICATION

(11) **EP 3 634 580 B1**
(45) Date of publication and mention of the grant of the patent: **20.12.2023**
(21) Application number: 18707937.1
(22) Date of filing: 07.03.2018
(51) Int. Cl.: A61K 31/14, A61P 1/00, A61P 19/00, A61P 17/00, A61K 31/203, A61K 31/375, A61K 31/66, A61K 31/7016, A61K 31/7135

(54) **COMPOSITIONS COMPRISING LACTULOSE FOR MAINTAINING OR IMPROVING GUT HEALTH IN HUMANS**
ZUSAMMENSETZUNGEN MIT LACTULOSE ZUR BEWAHRUNG ODER VERBESSERUNG DER DARMGESUNDHEIT BEI MENSCHEN
COMPOSITIONS CONTENANT DU LACTULOSE PERMETTANT DE MAINTENIR OU D'AMÉLIORER LA SANTÉ DE L'INTESTIN CHEZ L'HOMME

(30) Priority: 28.04.2017 EP 17168700
(43) Date of publication of application: 15.04.2020
(73) Proprietor: Fresenius Kabi Austria GmbH, 8055 Graz (AT)
(72) Inventor: KUCHINKA-KOCH, Angelika, 4020 Linz (AT)
(74) Representative: Fresenius Kabi Deutschland GmbH
(86) International application number: PCT/EP2018/055560
(87) International publication number: WO 2018/197087

(56) References cited:
- WO-A1-2012/149353
- CN-A- 102 871 020
- CN-A- 104 489 102
- JP-A- 2006 104 105
- US-A1- 2005 276 838

## Description

### Field of the invention

The invention relates to compositions comprising lactulose, one or more vitamins and, optionally, one or more minerals. These compositions can be used in maintaining or improving gut health in humans.

### Background of the invention

The microbiome of the human gastrointestinal tract mainly consists of the following four phyla: Bacteroidetes, Firmicutes, Actinobacter and Proteobacteria. The symbiotic relationship between a healthy microbiome and the host is essential to overall health.

One of the major functions of the gut microflora includes the development and homeostasis of the immune system. The microbiome has an important function to ensure normal permeability of the intestinal mucosa and mucosal integrity. Modifications to this intestinal integrity can be related to the development of systemic disorders. Alterations in the brain-gut microbiota interactions are believed to be involved in the pathogenesis of various disorders. These are, for example, irritable bowel syndrome and functional gastrointestinal disorders as well as brain disorders, such as autism spectrum disorders, Parkinson's disease and mood disorders.

Keeping the digestion in balance is crucial to being healthy and feeling well. Fibre is important for digestive health. There are two main groups of dietary fibre, soluble and insoluble. Insoluble fibre bulks up stools and makes waste move through the digestive tract more quickly, which is better for the gut. Soluble fibre helps to keep stools soft, which makes them easier to pass.

The bacteria in the gut are mainly in the colon. Soluble fibre is not digested in the small intestine. It passes through the small intestine unchanged and reaches the colon undigested. It is fermented by bacteria in the colon and, thus, supports the growth of gut bacteria. A diet rich in fibre provides the gut bacteria with material to grow on.

Sometimes it is hard to keep the digestion in balance. An unhealthy lifestyle, stress, travelling, pregnancy, or certain medical conditions can cause digestive irregularities. Furthermore, digestive irregularities can occur as side effect of certain pharmaceuticals. The prevalence of digestive irregularities increases with age. For example, digestive irregularities frequently occur in people over the age of 50.

Accordingly, there is a need for compositions which can be used in maintaining or improving gut health in humans. There is also a need for compositions which can additionally be used in at least one of maintaining or improving immune health, maintaining or improving skin health, maintaining or improving bone health, maintaining or improving liver health, protecting cells and maintaining or improving the gut-brain axis in humans. Preferably, such compositions are convenient to use, in particular easy to administer.

Oral compositions, in particular food supplements, comprising vitamins and/or minerals are known. Examples of commercial products include "Doppelherz aktiv Magnesium + B6 + B12 + Folsäure 400 Direct", "Sanostol Spezial Energie Sticks" and "dm Das gesunde Plus Magnesium Kalium Sticks direkt". These compositions do, however, not comprise lactulose.

In addition to vitamins and/or minerals such compositions typically comprise further ingredients, such as anti-caking agents, acidifiers, sweeteners, flavoring agents, fillers, thickeners, pH regulators, or the like. Examples of typical ingredients include magnesium salts, fatty acids, magnesium salts of fatty acids, magnesium oxide, silicon dioxide, citric acid, sodium citrate, trimagnesium dicitrate, sugar, sucralose, sorbitol, aspartame, acesulfame potassium, xylitol, mannitol, maltodextrin, carboxymethylcellulose sodium (carmellose sodium), cornstarch, potassium hydrogen carbonate, potassium hydrogen phosphate, coconut oil, or the like. Document JP2006104105 discloses a composition comprising lactulose 96-15% and calcium, plus optionally magnesium, and a vitamin D. In experiment 2, an amount of 75.5% lactulose is disclosed. It is used to improve bone health. The composition is solid (mix of powders) and lactulose is present in amount of up to 96%.

### Summary of the invention

The inventors have found that compositions comprising a) lactulose, b) at least one vitamin selected from vitamin A, vitamin C, vitamin D and vitamin E, and c) at least one member of the group consisting of calcium, phosphorus, zinc, selenium, and choline, preferably in the amounts specified herein, can be used in maintaining or improving gut health in humans. In addition, these compositions can be used in at least one of maintaining or improving immune health, maintaining or improving skin health, maintaining or improving bone health, maintaining or improving liver health, protecting cells and maintaining or improving the gut-brain axis in humans. One advantage of these compositions is that they are convenient to use, in particular easy to administer.

The compositions of the invention deliver health promoting ingredients focusing on health targets which are related to a balanced colonic flora. These are, for example, the immune system, skin and bones, the liver or the gut-brain axis.

Accordingly, in one aspect of the invention there is provided a composition comprising
a) 60-99 wt% of lactulose, based on the total weight of the composition;
b) at least one vitamin selected from vitamin A, vitamin C, vitamin D and vitamin E; and
c) at least one member of the group consisting of calcium, phosphorus, zinc, selenium, and choline, as defined in the claims.

In a further aspect of the invention there is provided a dose unit comprising the composition of the invention.

In a further aspect of the invention there is provided a dosage regime for use in maintaining or improving gut health in humans.

In a further aspect of the invention there is provided a composition as defined herein for use in maintaining or improving gut health in humans.

### Detailed description of the invention

The invention is defined by the claims. Any subject-matter falling outside the scope of the claims is provided for information purposes only.

The composition of the invention is an enteral composition, in particular an oral composition. Preferably, the composition of the invention is a food supplement.

The composition of the invention is in the form of a solid. For example, the composition of the invention can be in the form of a powder, granules, crystals, or mixtures thereof.

According to preferred embodiments, the composition of the invention is a composition which is in the form of a solid and which can be administered without using any additional water. This means that such a composition does not need to be dissolved or suspended in a liquid, such as water, prior to administration. Instead, it can be administered in the form of a solid. Moreover, there is no need to drink, for example, water after administration. Such a composition directly dissolves in the mouth, e.g. on the tongue. If desired, one can, of course, drink water after administration. However, as mentioned above, there is no need to do so.

According to also preferred embodiments, the composition of the invention is a composition which is administered with the use of additional water. Such a composition is typically dissolved or suspended in a liquid, such as water, prior to administration.

The present invention covers both compositions which can be administered without using any additional water and compositions which are administered with the use of additional water.

In preferred embodiments at least 60 wt%, preferably at least 65 wt%, more preferably at least 70%, even more preferably at least 75 wt%, particularly preferably at least 80 wt% of the particles present in the composition have a particle size ≤ 500 µm, preferably ≤ 450 µm, more preferably ≤ 420 µm.

In further preferred embodiments at least 60 wt%, preferably at least 65 wt%, more preferably at least 70%, even more preferably at least 75 wt%, particularly preferably at least 80 wt% of the particles present in the composition have a particle size in the range of from 100 to 500 µm, preferably from 100 to 450 µm, more preferably from 100 to 400 µm.

The particle size distribution of the particles present in the composition can be determined by using sieves having different mesh sizes. A person skilled in the art knows how to do that.

Preferably, the total weight of the composition of the invention is 1.9-3.9 g, preferably 2.0-3.7 g, more preferably 2.1-3.5 g. In a preferred embodiment the total weight of the composition of the invention is 1.9-2.5 g, preferably 2.0-2.4 g, more preferably 2.1-2.3 g. In another preferred embodiment the total weight of the composition of the invention is 2.6-3.5 g, preferably 2.8-3.3 g, more preferably 2.9-3.1 g. In another preferred embodiment the total weight of the composition of the invention is 3.0-3.9 g, preferably 3.2-3.7 g, more preferably 3.3-3.5 g.

Also preferably, the total weight of the composition of the invention is 1.9-6.5 g, preferably 2.0-6.0 g, more preferably 2.1-5.2 g. In another preferred embodiment the total weight of the composition of the invention is 2.6-6.5 g, preferably 2.8-6.0 g, more preferably 2.9-5.2 g. In another preferred embodiment the total weight of the composition of the invention is 3.0-6.5 g, preferably 3.2-6.0 g, more preferably 3.3-5.2 g.

Preferably, the composition of the invention comprises 1.8-3.6 g, preferably 1.9-3.4 g, more preferably 2.0-3.2 g of lactulose. In a preferred embodiment the composition of the invention comprises 1.8-2.4 g, preferably 1.9-2.3 g, more preferably 2.0-2.2 g of lactulose. In another preferred embodiment the composition of the invention comprises 1.8-2.7 g, preferably 1.9-2.5 g, more preferably 2.0-2.3 g of lactulose. In another preferred embodiment the composition of the invention comprises 2.7-3.6 g, preferably 2.8-3.4 g, more preferably 2.9-3.2 g of lactulose.

Also preferably, the composition of the invention comprises 1.8-5.5 g, preferably 1.9-5.2 g, more preferably 2.0-4.5 g of lactulose. In another preferred embodiment the composition of the invention comprises 2.7-5.5 g, preferably 2.8-5.2 g, more preferably 2.9-4.5 g of lactulose.

The composition of the invention is preferably provided in package. For example, the composition of the invention can be provided in a bottle, tetra brick, bag, sachet, stick, or the like.

### Lactulose

Lactulose is a disaccharide, which is not digested in the small intestine. It passes through the small intestine unchanged and reaches the colon undigested. It is fermented by bacteria in the colon and, thus, supports the growth of gut bacteria. Therefore, lactulose represents an example of soluble fiber and is also prebiotic.

The composition of the invention comprises lactulose. The lactulose is in the form of a solid. Preferably, the lactulose is crystalline. In a preferred embodiment the lactulose is present in the form of lactulose crystals. Herein the terms "crystalline lactulose" and "lactulose crystals" are used interchangeably.

In a preferred embodiment at least 75 wt%, preferably at least 80 wt%, more preferably at least 85%, even more preferably at least 88 wt% of the lactulose has a particle size ≤ 500 µm, preferably ≤ 450 µm, more preferably ≤ 420 µm, as far as falling under the presently claimed range.

According to the present invention, at least 88 wt% of the lactulose has a particle size in the range of from 100 to 400 µm.

In another embodiment at least 80 wt%, preferably at least 85 wt%, more preferably at least 90 wt% of the lactulose has a particle size < 250 µm, preferably < 200 µm, more preferably < 150 µm, as far as falling under the presently claimed range.

The particle size distribution of the lactulose can be determined by using sieves having different mesh sizes. A person skilled in the art knows how to do that.

The composition of the invention comprises at least 60 wt% of lactulose, based on the total weight of the composition. In a preferred embodiment the composition of the invention comprises at least 75 wt% of lactulose, based on the total weight of the composition. In another preferred embodiment the composition of the invention comprises at least 80 wt% of lactulose, based on the total weight of the composition.

Preferably, the composition of the invention comprises 60-99 wt%, more preferably 65-98 wt%, even more preferably 70-96 wt% of lactulose, based on the total weight of the composition.

In a preferred embodiment the composition of the invention comprises 85-99 wt%, preferably 90-98 wt%, more preferably 92-96 wt% of lactulose, based on the total weight of the composition.

In another preferred embodiment the composition of the invention comprises 60-90 wt%, preferably 65-80 wt%, more preferably 70-75 wt% of lactulose, based on the total weight of the composition.

In another preferred embodiment the composition of the invention comprises 80-99 wt%, preferably 85-95 wt%, more preferably 88-92 wt% of lactulose, based on the total weight of the composition.

Lactulose can, for example, be prepared from lactose by isomerization. Lactulose crystals are commercially available from Fresenius Kabi.

Lactulose helps to protect and promote growth of gut bacteria, support a healthy gut flora and accelerate intestinal transit time. Furthermore, lactulose acts as a detox agent, e.g. by supporting bowel movements. This is important because waste and toxic substances are eliminated from the body by the liver into the gut and are further excreted with the stool.

### Vitamins

The composition of the invention comprises at least one vitamin. Preferably, the composition of the invention comprises at least one vitamin selected from vitamin A, vitamin B, vitamin C, vitamin D, vitamin E and vitamin K.

In a preferred embodiment the composition of the invention comprises at least one vitamin selected from vitamin A, vitamin D, vitamin E and vitamin K. In a more preferred embodiment the composition of the invention comprises at least one vitamin selected from vitamin A, vitamin D and vitamin E. In a further embodiment the composition of the invention comprises vitamin K.

In another preferred embodiment the composition of the invention comprises at least one vitamin selected from vitamin C and vitamin B. In a more preferred embodiment the composition of the invention comprises vitamin C. In a further embodiment the composition of the invention comprises vitamin B.

In a particularly preferred embodiment the composition of the invention comprises at least one vitamin selected from vitamin A, vitamin C, vitamin D and vitamin E.

Vitamin A is important for the growth, function and structure of the skin and mucous membranes, for the formation of blood cells, for metabolism and for the optic nerve. Vitamin A also increases the resistance to infections.

Vitamin C is an antioxidant, which stimulates the immune system. Vitamin E is an antioxidant, which reduces the risk or impact of lipid peroxidation.

In a preferred embodiment the composition of the invention comprises at least one vitamin selected from vitamin A and vitamin C. In another preferred embodiment the composition of the invention comprises vitamin A. In another preferred embodiment the composition of the invention comprises vitamin C. In a further preferred embodiment the composition of the invention comprises vitamin A and vitamin C.

In a preferred embodiment the composition of the invention comprises vitamin D. The term "vitamin D" as used herein preferably refers to vitamin D₃.

In a preferred embodiment the composition of the invention comprises vitamin E.

If present, the composition of the invention comprises, for example, 90-545 µg RE, preferably 90-220 µg RE, more preferably 100-200 µg RE, even more preferably 115-188 µg RE vitamin A per g of the composition.

If present, the composition of the invention comprises, for example, at least 90 µg RE, preferably 90-160 µg RE, more preferably 100-140 µg RE, even more preferably 115-135 µg RE vitamin A per g of the composition.

If present, the composition of the invention comprises, for example, 150-545 µg RE, preferably 150-220 µg RE, more preferably 165-200 µg RE, even more preferably 176-188 µg RE vitamin A per g of the composition. The expression "RE" as used herein refers to retinol equivalents.

If present, the composition of the invention comprises, for example, at least 10 mg, preferably 10-55 mg, more preferably 15-45 mg, even more preferably 20-38 mg vitamin C per g of the composition.

If present, the composition of the invention comprises, for example, at least 10 mg, preferably 10-40 mg, more preferably 15-35 mg, even more preferably 20-30 mg vitamin C per g of the composition.

If present, the composition of the invention comprises, for example, at least 20 mg, preferably 20-55 mg, more preferably 30-45 mg, even more preferably 35-38 mg vitamin C per g of the composition.

If present, the composition of the invention comprises, for example, at least 1.1 µg, preferably 1.1-2.5 µg, more preferably 1.4-2.1 µg, even more preferably 1.6-1.8 µg vitamin D per g of the composition.

If present, the composition of the invention comprises, for example, 1.0-5.5 mg, preferably 1.0-2.6 mg, more preferably 1.1-2.2 mg, even more preferably 1.2-1.9 mg alpha-TE vitamin E per g of the composition.

If present, the composition of the invention comprises, for example, at least 1.0 mg, preferably 1.0-2.0 mg, more preferably 1.1-1.8 mg, even more preferably 1.2-1.6 mg alpha-TE vitamin E per g of the composition.

If present, the composition of the invention comprises, for example, 1.1-5.5 mg, preferably 1.1-2.6 mg, more preferably 1.4-2.2 mg, even more preferably 1.6-1.9 mg alpha-TE vitamin E per g of the composition. The expression "alpha-TE" as used herein refers to alpha-tocopherol equivalents.

### Minerals

Optionally, the composition of the invention comprises one or more minerals. Preferably, the composition of the invention comprises one or more minerals selected from sodium, chloride, potassium, calcium, phosphorus, magnesium, iron, zinc, copper, iodine, selenium, manganese, chromium, molybdenum and fluoride.

In a preferred embodiment the composition of the invention comprises one or more minerals selected from sodium, potassium, calcium, phosphorus, magnesium, iron, zinc, copper, selenium, manganese, chromium and molybdenum. In a further embodiment the composition of the invention comprises one or more minerals selected from chloride, iodine and fluoride.

In a particularly preferred embodiment the composition of the invention comprises one or more minerals selected from calcium, phosphorus, zinc and selenium.

Zinc plays a key role in carbohydrate, lipid and protein metabolism and contributes to the formation of genetic material and cell growth. Selenium has immunomodulatory effects.

In a preferred embodiment the composition of the invention comprises selenium.

In a preferred embodiment the composition of the invention comprises at least one mineral selected from calcium, phosphorus and zinc. In another preferred embodiment the composition of the invention comprises calcium. In another preferred embodiment the composition of the invention comprises phosphorus. In another preferred embodiment the composition of the invention comprises zinc. In a further preferred embodiment the composition of the invention comprises calcium and phosphorus. In a further preferred embodiment the composition of the invention comprises calcium, phosphorus and zinc.

Calcium can, for example, be present in the form of one or more calcium salts, preferably calcium(II) salts. Suitable calcium salts are known to a person skilled in the art. Preferred calcium salts are selected from dicalcium phosphate, calcium phosphate, calcium acetate, calcium chloride, calcium gluconate, calcium ascorbate, calcium lactate, and mixtures thereof. A particularly preferred calcium salt is dicalcium phosphate (calcium hydrogen phosphate).

If present, the composition of the invention comprises, for example, 40-400 mg, preferably 40-70 mg, more preferably 48-62 mg, even more preferably 52-56 mg calcium per g of the composition.

Phosphorus can, for example, be present in the form of one or more phosphorus compounds. Suitable phosphorus compounds are known to a person skilled in the art. Preferred phosphorus compounds are selected from dicalcium phosphate, calcium phosphate, dimagnesium phosphate, magnesium phosphate, disodium phosphate, sodium phosphate, dipotassium phosphate, potassium phosphate, and mixtures thereof. A particularly preferred phosphorus compound is dicalcium phosphate (calcium hydrogen phosphate).

If present, the composition of the invention comprises, for example, 25-340 mg, preferably 25-60 mg, more preferably 35-50 mg, even more preferably 40-43 mg phosphorus per g of the composition.

Zinc can, for example, be present in the form of one or more zinc salts, preferably zinc(ll) salts. Suitable zinc salts are known to a person skilled in the art. Preferred zinc salts are selected from zinc citrate, zinc acetate, zinc chloride, zinc gluconate, zinc sulfate, zinc carbonate, zinc oxide, and mixtures thereof. A particularly preferred zinc salt is zinc citrate.

If present, the composition of the invention comprises, for example, at least 2.5 mg, preferably 2.5-4.8 mg, more preferably 2.8-4.0 mg, even more preferably 3.2-3.5 mg zinc per g of the composition.

Selenium can, for example, be present in the form of one or more selenium compounds. Suitable selenium compounds are known to a person skilled in the art. Preferred selenium compounds are selected from sodium selenate, sodium selenite, selenomethionine, and mixtures thereof. A particularly preferred selenium compound is sodium selenate.

If present, the composition of the invention comprises, for example, at least 10 µg, preferably 10-40 µg, more preferably 12-30 µg, even more preferably 15-26 µg selenium per g of the composition.

If present, the composition of the invention comprises, for example, at least 10 µg, preferably 10-30 µg, more preferably 12-24 µg, even more preferably 15-20 µg selenium per g of the composition.

If present, the composition of the invention comprises, for example, at least 15 µg, preferably 15-40 µg, more preferably 20-30 µg, even more preferably 24-26 µg selenium per g of the composition.

### Choline

Optionally, the composition of the invention comprises choline. Choline, which refers to (2-hydroxyethyl)trimethylammonium, is a quaternary ammonium ion. Thus, choline is typically present in the form of one or more choline salts. Suitable choline salts are known to a person skilled in the art. Preferred choline salts are selected from choline bitartrate, choline acetate, choline chloride, choline para-toluenesulfonate, choline bicarbonate, choline hydroxide, choline dihydrogencitrate, tricholine citrate, and mixtures thereof. A particularly preferred choline salt is choline bitartrate.

If present, the composition of the invention comprises, for example, at least 12 mg, preferably 12-118 mg, more preferably 15-30 mg, even more preferably 17-26 mg choline per g of the composition.

If present, the composition of the invention comprises, for example, at least 12 mg, preferably 12-50 mg, more preferably 15-35 mg, even more preferably 17-24 mg choline per g of the composition.

If present, the composition of the invention comprises, for example, at least 15 mg, preferably 15-118 mg, more preferably 20-30 mg, even more preferably 23-26 mg choline per g of the composition. The amounts specified herein for choline refer to choline as such, i.e. the (2-hydroxyethyl)trimethylammonium ion (not to the respective choline salt, i.e. choline + its counterion).

### Further components and additives

Optionally, the composition of the invention comprises one or more anti-caking agents. Suitable anti-caking agents are known to a person skilled in the art. Preferred anti-caking agents are selected from silicon dioxide, sodium silicate, magnesium trisilicate, aluminum silicate, sodium aluminosilicate, potassium aluminum silicate, fatty acids (such as stearic acid), magnesium salts of fatty acids (such as magnesium stearate), and mixtures thereof. Particularly preferred anti-caking agents are selected from silicon dioxide, magnesium salts of fatty acids (such as magnesium stearate), and mixtures thereof. Particular preference is given to silicon dioxide. Also particular preference is given to magnesium salts of fatty acids (such as magnesium stearate). In a particular embodiment the composition of the invention comprises silicon dioxide and magnesium salts of fatty acids (such as magnesium stearate).

If present, the composition of the invention comprises, for example, 0.05-1.80 wt%, preferably 0.10-1.25 wt%, more preferably 0.13-1.00 wt% of one or more anti-caking agents, based on the total weight of the composition.

If present, the composition of the invention comprises, for example, 0.40-1.80 wt%, preferably 0.45-1.25 wt%, more preferably 0.50-1.00 wt% of one or more anti-caking agents, based on the total weight of the composition.

If present, the composition of the invention comprises, for example, 0.1-1.5 wt%, preferably 0.2-1.0 wt%, more preferably 0.3-0.8 wt% of silicon dioxide, based on the total weight of the composition.

If present, the composition of the invention comprises, for example, 0.05-0.30 wt%, preferably 0.10-0.25 wt%, more preferably 0.13-0.20 wt% of magnesium salts of fatty acids, based on the total weight of the composition.

Optionally, the composition of the invention comprises one or more acidifiers. Suitable acidifiers are known to a person skilled in the art. Preferred acidifiers are selected from citric acid, formic acid, propionic acid, lactic acid, fumaric acid, phosphoric acid, and mixtures thereof. A particularly preferred acidifier is citric acid.

If present, the composition of the invention comprises, for example, 0.4-5.0 wt%, preferably 0.6-4.0 wt%, more preferably 0.8-3.0 wt% of one or more acidifiers, based on the total weight of the composition.

If present, the composition of the invention comprises, for example, 0.5-5.0 wt%, preferably 1.0-4.0 wt%, more preferably 1.2-3.0 wt% of one or more acidifiers, based on the total weight of the composition.

Optionally, the composition of the invention comprises one or more sweeteners. Suitable sweeteners are known to a person skilled in the art. Preferred sweeteners are selected from sucralose, sorbitol, aspartame, and mixtures thereof. A particularly preferred sweetener is sucralose.

If present, the composition of the invention comprises, for example, 0.02-0.20 wt%, preferably 0.05-0.15 wt%, more preferably 0.08-0.12 wt% of one or more sweeteners, based on the total weight of the composition.

Optionally, the composition of the invention comprises one or more flavoring agents. Suitable flavoring agents are known to a person skilled in the art. Natural as well as artificial flavors are suitable. Examples of preferred flavors are ginger flavor, apple flavor, cherry flavor, sour cherry flavor, orange flavor, lemon flavor, grapefruit flavor, pineapple flavor, vanilla flavor, caramel flavor, chocolate flavor, hazelnut flavor, or the like. For example, the composition of the invention has a ginger flavor, apple flavor or sour cherry flavor. In a particular embodiment the composition of the invention comprises ginger extract.

If present, the composition of the invention comprises, for example, 0.2-2.0 wt%, preferably 0.4-1.5 wt%, more preferably 0.6-1.2 wt% of one or more flavoring agents, based on the total weight of the composition.

### Dose unit and dosage regime

The composition of the invention can, for example, be provided in a dose unit. In one aspect of the invention there is provided a dose unit comprising the composition of the invention.

Such a dose unit is preferably provided in package. For example, such a dose unit can be provided in a bottle, tetra brick, bag, sachet, stick, or the like.

Preferably, such a dose unit provides 1.9-3.9 g, preferably 2.0-3.7 g, more preferably 2.1-3.5 g of the composition of the invention. In a preferred embodiment such a dose unit provides 1.9-2.5 g, preferably 2.0-2.4 g, more preferably 2.1-2.3 g of the composition of the invention. In another preferred embodiment such a dose unit provides 2.6-3.5 g, preferably 2.8-3.3 g, more preferably 2.9-3.1 g of the composition of the invention. In another preferred embodiment such a dose unit provides 3.0-3.9 g, preferably 3.2-3.7 g, more preferably 3.3-3.5 g of the composition of the invention.

Also preferably, such a dose unit provides 1.9-6.5 g, preferably 2.0-6.0 g, more preferably 2.1-5.2 g of the composition of the invention. In another preferred embodiment such a dose unit provides 2.6-6.5 g, preferably 2.8-6.0 g, more preferably 2.9-5.2 g of the composition of the invention. In another preferred embodiment such a dose unit provides 3.0-6.5 g, preferably 3.2-6.0 g, more preferably 3.3-5.2 g of the composition of the invention.

Preferably, such a dose unit provides 1.8-3.6 g, preferably 1.9-3.4 g, more preferably 2.0-3.2 g of lactulose. In a preferred embodiment such a dose unit provides 1.8-2.4 g, preferably 1.9-2.3 g, more preferably 2.0-2.2 g of lactulose. In another preferred embodiment such a dose unit provides 1.8-2.7 g, preferably 1.9-2.5 g, more preferably 2.0-2.3 g of lactulose. In another preferred embodiment such a dose unit provides 2.7-3.6 g, preferably 2.8-3.4 g, more preferably 2.9-3.2 g of lactulose.

Also preferably, such a dose unit provides 1.8-5.5 g, preferably 1.9-5.2 g, more preferably 2.0-4.5 g of lactulose. In another preferred embodiment such a dose unit provides 2.7-5.5 g, preferably 2.8-5.2 g, more preferably 2.9-4.5 g of lactulose.

In a particularly preferred embodiment such a dose unit provides
b1) 330-484 µg RE, preferably 363-440 µg RE, more preferably 387-414 µg RE vitamin A;
b2) 44-121 mg, preferably 66-99 mg, more preferably 77-84 mg vitamin C; and
c4) 33-88 µg, preferably 44-66 µg, more preferably 53-57 µg selenium.

In another particularly preferred embodiment such a dose unit provides
b3) 3.3-7.5 µg, preferably 4.2-6.3 µg, more preferably 4.8-5.4 µg vitamin D;
c1) 120-210 mg, preferably 144-186 mg, more preferably 156-168 mg calcium;
c2) 75-180 mg, preferably 105-150 mg, more preferably 120-129 mg phosphorus; and
c3) 7.5-14.5 mg, preferably 8.4-12.0 mg, more preferably 9.6-10.5 mg zinc.

In another particularly preferred embodiment such a dose unit provides
b4) 3.8-8.6 mg, preferably 4.8-7.5 mg, more preferably 5.6-6.4 mg alpha-TE vitamin E; and
c5) 60-400 mg, preferably 70-100 mg, more preferably 78-88 mg choline.

A typical daily dose of the composition of the invention can, for example, be provided by 1 to 3, preferably 1 or 2, particularly preferably 1 dose unit as defined herein. Accordingly, a typical weekly dose of the composition of the invention can, for example, be provided by 7 to 21, preferably 7 to 14, particularly preferably 7 dose units as defined herein.

Another typical weekly dose of the composition of the invention can, for example, be provided by 1 to 10, preferably 1 to 7, more preferably 1 to 4, particularly preferably 1 or 2, also particularly preferably 3 or 4 dose units as defined herein.

In one aspect of the invention there is provided a dosage regime for use in maintaining or improving gut health in humans, wherein a daily dose is provided by 1 to 3, preferably 1 or 2, particularly preferably 1 dose unit as defined herein.

In another aspect of the invention there is provided a dosage regime for use in maintaining or improving gut health in humans, wherein a weekly dose is provided by 1 to 10, preferably 1 to 7, more preferably 1 to 4, particularly preferably 1 or 2, also particularly preferably 3 or 4 dose units as defined herein.

### Use of the composition of the invention

The composition of the invention can, for example, be used in maintaining or improving gut health in humans. Accordingly, in one aspect of the invention there is provided a composition as defined herein for use in maintaining or improving gut health in humans. Preferably, the composition of the invention is used in maintaining or improving gut health and at least one of maintaining or improving immune health, maintaining or improving skin health, maintaining or improving bone health, maintaining or improving liver health and protecting cells in humans. Also preferably, the composition of the invention is used in maintaining or improving gut health and maintaining or improving the gut-brain axis in humans.

In a particular embodiment the composition of the invention is used in maintaining or improving gut health and immune health in humans.

In a further particular embodiment the composition of the invention is used in maintaining or improving gut health and at least one of skin health and bone health in humans, preferably maintaining or improving gut health, skin health and bone health in humans.

In a further particular embodiment the composition of the invention is used in maintaining or improving gut health and at least one of maintaining or improving liver health and protecting cells in humans, preferably maintaining or improving gut health and liver health and protecting cells in humans.

The expression "maintaining or improving gut health" as used herein preferably means protecting gut bacteria, promoting growth of gut bacteria, supporting a healthy gut flora and/or accelerating intestinal transit time. The expression "maintaining or improving immune health" as used herein preferably means maintenance of the normal function of the immune system. The expression "maintaining or improving skin health" as used herein preferably means maintenance of normal skin. The expression "maintaining or improving bone health" as used herein preferably means maintenance of normal bones. The expression "maintaining or improving liver health" as used herein preferably means maintenance of normal liver function. The expression "protecting cells" as used herein preferably means protection of cells from oxidative stress.

The expression "humans" as used herein includes both males and females. They can be at any age. Preferably, they are 3 years or older, more preferably 14 years or older, even more preferably 18 years or older. For example, they can be 25 years or older, 30 years or older, 40 years or older, 50 years or older, 60 years or older, or 70 years or older. Particularly preferably, they are 50 years or older.

According to a particular aspect, the invention relates to lactulose, vitamin A, vitamin C and selenium for use in maintaining or improving gut health in humans (preferably for use in maintaining or improving gut health and immune health in humans), wherein said active ingredients are administered in the form of a composition comprising lactulose, vitamin A, vitamin C and selenium, preferably in the amounts specified herein.

Vitamin A, vitamin C and selenium contribute to the normal function of the immune system.

According to a further particular aspect, the invention relates to lactulose, vitamin D, calcium, phosphorus and zinc for use in maintaining or improving gut health in humans (preferably for use in maintaining or improving gut health and at least one of (preferably both) skin health and bone health in humans), wherein said active ingredients are administered in the form of a composition comprising lactulose, vitamin D, calcium, phosphorus and zinc, preferably in the amounts specified herein.

Zinc contributes to the maintenance of normal skin. Calcium and phosphorus contribute to the maintenance of normal bones. Vitamin D contributes to normal utilization of calcium and phosphorus. Lactulose enhances the absorption of calcium.

According to a further particular aspect, the invention relates to lactulose, vitamin E and choline for use in maintaining or improving gut health in humans (preferably for use in maintaining or improving gut health and at least one of (preferably both) maintaining or improving liver health and protecting cells in humans), wherein said active ingredients are administered in the form of a composition comprising lactulose, vitamin E and choline, preferably in the amounts specified herein.

Vitamin E contributes to the protection of cells from oxidative stress. Choline contributes to the maintenance of normal liver function. Choline also acts as a lipotropic factor; it helps the liver to process lipids and, thus, to prevent accumulation of lipids in the liver.

The composition of the invention is administered enterally, in particular orally.

Preferably, the composition of the invention is administered in the form of a solid. For example, the composition of the invention can be administered in the form of a powder, granules, crystals, or mixtures thereof.

Preferably, the composition is provided in a dose unit of 1.9-3.9 g, preferably 2.0-3.7 g, more preferably 2.1-3.5 g. Also preferably, the composition is provided in a dose unit of 1.9-6.5 g, preferably 2.0-6.0 g, more preferably 2.1-5.2 g.

Preferably, the composition is provided in a dose unit providing 1.8-3.6 g, preferably 1.9-3.4 g, more preferably 2.0-3.2 g of lactulose. Also preferably, the composition is provided in a dose unit providing 1.8-5.5 g, preferably 1.9-5.2 g, more preferably 2.0-4.5 g of lactulose.

Preferably, the composition is administered in 1 to 3, preferably 1 or 2, particularly preferably 1 dose unit as defined herein daily.

Preferably, the composition is administered in 1 to 10, preferably 1 to 7, more preferably 1 to 4, particularly preferably 1 or 2, also particularly preferably 3 or 4 dose units as defined herein weekly.

### Preparation of the composition of the invention

The composition of the invention can be prepared according to methods known in the art. For example, the composition of the invention can be prepared by mixing its components. Suitable mixing techniques are known to a person skilled in the art.

The invention is further illustrated by the following examples.

### Examples

### Example 1

A composition for maintaining or improving gut health in humans (preferably for maintaining or improving gut health and immune health in humans) was prepared by mixing its components. The composition (total weight: 2.2 g) comprises the components listed in the following Table:

| **Component** | **Amount** |
|---|---|
| Crystalline lactulose* | 2.07 g (94.1 wt%) |
| Vitamin A (retinyl acetate) | 400 µg RE |
| Vitamin C (L-ascorbic acid) | 80 mg |
| Selenium (added in the form of sodium selenate) | 55 µg selenium |
| Silicon dioxide (anti-caking agent) | 0.5 wt% |
| Flavoring / aroma | 1.0 wt% |
| Ginger extract + silicon dioxide + maltodextrin | 0.03 wt% |

| | |
|---|---|
| * 90 wt% of the crystalline lactulose used in this example has a particle size in the range of from 100 to 400 µm | |

The composition is provided in package, more specifically in a stick. The composition is in the form of a solid and can be administered without using any additional water. The composition directly dissolves in the mouth, e.g. on the tongue.

### Example 2

A composition for maintaining or improving gut health in humans (preferably for maintaining or improving gut health, skin health and bone health in humans) was prepared by mixing its components. The composition (total weight: 3.0 g) comprises the components listed in the following Table:

| **Component** | **Amount** |
|---|---|
| Crystalline lactulose* | 2.14 g (71.3 wt%) |
| Vitamin D₃ (cholecalciferol) | 5 µg |
| Calcium (added in the form of dicalcium phosphate) | 160 mg calcium |
| Phosphorus (added in the form of dicalcium phosphate) | 123.5 mg phosphorus |
| Zinc (added in the form of zinc citrate) | 10 mg zinc |
| Silicon dioxide (anti-caking agent) | 0.4 wt% |
| Magnesium salts of fatty acids (anti-caking agent) | 0.16 wt% |
| Citric acid (acidifier) | 2.7 wt% |
| Sucralose (sweetener) | 0.1 wt% |
| Flavoring / aroma | 0.7 wt% |

| | |
|---|---|
| * 90 wt% of the crystalline lactulose used in this example has a particle size in the range of from 100 to 400 µm | |

The composition is provided in package, more specifically in a stick. The composition is in the form of a solid and can be administered without using any additional water. The composition directly dissolves in the mouth, e.g. on the tongue.

### Example 3

A composition for maintaining or improving gut health in humans (preferably for maintaining or improving gut health and liver health and protecting cells in humans) was prepared by mixing its components. The composition (total weight: 3.4 g) comprises the components listed in the following Table:

| **Component** | **Amount** |
|---|---|
| Crystalline lactulose* | 3.06 g (90 wt%) |
| Vitamin E (DL-alpha-tocopheryl acetate) | 6 mg alpha-TE |
| Choline (added in the form of choline bitartrate) | 82.5 mg choline |
| Citric acid (acidifier) | 1.5 wt% |
| Flavoring / aroma | 1.0 wt% |
| Silicon dioxide (anti-caking agent) | 0.7 wt% |
| Magnesium salts of fatty acids (anti-caking agent) | 0.16 wt% |

| | |
|---|---|
| * 90 wt% of the crystalline lactulose used in this example has a particle size in the range of from 100 to 400 µm | |

The composition is provided in package, more specifically in a stick. The composition is in the form of a solid and can be administered without using any additional water. The composition directly dissolves in the mouth, e.g. on the tongue.

### Example 4

A composition for maintaining or improving gut health in humans (preferably for maintaining or improving gut health and immune health in humans) was prepared by mixing its components. The composition (total weight: 3.2 g) comprises the components listed in the following Table:

| **Component** | **Amount** |
|---|---|
| Crystalline lactulose* | 3.0 g (94 wt%) |
| Vitamin A (retinyl acetate) | 400 µg RE |
| Vitamin C (L-ascorbic acid) | 80 mg |
| Selenium (added in the form of sodium selenate) | 55 µg selenium |
| Silicon dioxide (anti-caking agent) | 0.5 wt% |
| Flavoring / aroma | 0.9 wt% |
| Ginger extract + silicon dioxide + maltodextrin | 0.02 wt% |
| Citric acid (acidifier) | 1.0 wt% |

| | |
|---|---|
| * 90 wt% of the crystalline lactulose used in this example has a particle size in the range of from 100 to 400 µm | |

The composition is provided in package, more specifically in a stick. The composition is in the form of a solid and can be administered without using any additional water. The composition directly dissolves in the mouth, e.g. on the tongue.

### Example 5

A composition for maintaining or improving gut health in humans (preferably for maintaining or improving gut health and liver health and protecting cells in humans) was prepared by mixing its components. The composition (total weight: 4.4 g) comprises the components listed in the following Table:

| **Component** | **Amount** |
|---|---|
| Crystalline lactulose* | 4.0 g (91 wt%) |
| Vitamin E (DL-alpha-tocopheryl acetate) | 6 mg alpha-TE |
| Choline (added in the form of choline bitartrate) | 82.5 mg choline |
| Citric acid (acidifier) | 1.2 wt% |
| Flavoring / aroma | 0.8 wt% |
| Silicon dioxide (anti-caking agent) | 0.7 wt% |
| Magnesium salts of fatty acids (anti-caking agent) | 0.16 wt% |

| | |
|---|---|
| * 90 wt% of the crystalline lactulose used in this example has a particle size in the range of from 100 to 400 µm | |

The composition is provided in package, more specifically in a stick. The composition is in the form of a solid and can be administered without using any additional water. The composition directly dissolves in the mouth, e.g. on the tongue.

### Example 6

A composition (total weight: 2.2 g) comprising 2.07 g of crystalline lactulose, 400 µg RE vitamin A (retinyl acetate), 80 mg vitamin C (L-ascorbic acid) and 55 µg selenium was prepared by mixing its components. The composition was packed in a stick and then stored at room temperature for 14 months. After storage, the content of vitamin A, vitamin C and selenium in the composition was analyzed. The results of this stability test are shown in the following Table:

| **Component** | **Content* before storage** (calculated) | **Content* after storage** (analyzed) |
|---|---|---|
| Vitamin A | 18.2 mg | 19.8 mg |
| Vitamin C | 3.6 g | 3.5 g |
| Selenium | 2.5 mg | 2.3 mg |

| | | |
|---|---|---|
| * in 100 g of the composition | | |

These results demonstrate that the composition is stable at room temperature for at least 14 months.

### Example 7

A composition (total weight: 3.0 g) comprising 2.14 g of crystalline lactulose, 160 mg calcium, 123.5 mg phosphorus, 10 mg zinc and 5 µg vitamin D₃ (cholecalciferol) was prepared by mixing its components. The composition was packed in a stick and then stored at room temperature for 11 months. After storage, the content of calcium, phosphorus, zinc and vitamin D₃ in the composition was analyzed. The results of this stability test are shown in the following Table:

| **Component** | **Content* before storage** (calculated) | **Content* after storage** (analyzed) |
|---|---|---|
| Calcium | 5.3 g | 5.1 g |
| Phosphorus | 4.1 g | 4.5 g |
| Zinc | 333 mg | 346 mg |
| Vitamin D₃ | 167 µg | 179 µg |

| | | |
|---|---|---|
| * in 100 g of the composition | | |

These results demonstrate that the composition is stable at room temperature for at least 11 months.

### Example 8

A composition (total weight: 3.4 g) comprising 3.06 g of crystalline lactulose, 6 mg alpha-TE vitamin E (DL-alpha-tocopheryl acetate) and 82.5 mg choline was prepared by mixing its components. The composition was packed in a stick and then stored at room temperature for 11 months. After storage, the content of vitamin A, vitamin C and selenium in the composition was analyzed. The results of this stability test are shown in the following Table:

| **Component** | **Content* before storage** (calculated) | **Content* after storage** (analyzed) |
|---|---|---|
| Vitamin E | 176 mg | 182 mg |
| Choline | 2.4 g | 2.3 g |

| | | |
|---|---|---|
| * in 100 g of the composition | | |

These results demonstrate that the composition is stable at room temperature for at least 11 months.

### Example 9

Compositions according to Examples 4 and 5 were subjected to a taste and mouthfeel test (11 participants). All participants ingested the compositions without using any additional water and confirmed that the compositions dissolve fast when put in the mouth. Taste and mouthfeel were rated at least as good by all participants.

### Example 10

Crystalline lactulose was administered daily for 5 days to the TNO Intestinal Model (TIM-2), an in vitro model of the proximal colon. NaOH consumption, short-chain fatty acids (SCFA) production, ammonia production as well as relative abundance of microbiota were analyzed.

### 10.1 Materials and Methods

### Test Product:

The substance administered in this study was lactulose crystals (S.C.M. Società Chimica Mugello S.r.l., Vicchio, Italy). Experiments without the addition of lactulose served as negative control.

### Intestinal Conditions of the TIM-2 System:

The TNO Intestinal Model (TIM-2) is a dynamic in vitro model of the proximal colon. The TIM-2 system was inoculated with a highly metabolically active colon microbiota of human origin. In the system the following standardized conditions were simulated: body temperature; pH in the lumen of the proximal colon (pH 5.8); delivery of a substrate from the 'ileum' (SIEM; Standardized Ileum Efflux Medium); mixing and transport of the intestinal contents; anaerobiosis; absorption of water and absorption of fermentation products, metabolites and other low molecular weight compounds (via a semipermeable membrane inside the colon model).

SIEM simulates material passing the ileocecal valve in humans. It contained the major non-digestible carbohydrates (pectin, xylan, arabinogalactan, amylopectin, starch) found in a normal western diet as well as protein (bactopepton, casein), ox-bile, Tween 80 as well as vitamins and minerals. SIEM was added to the system at a speed of 2.5 mL/h and the speed of the dialysis liquid was 1.5 mL/min. During the experiment, the intestinal contents were mixed continuously by the peristaltic movements of the TIM-2 system. In order to simulate the transit of the chime from proximal to distal colon, 25 mL of the lumen was removed every 24 h and discarded.

Before performing each experiment, the system was inoculated with a standardized microbiota of human origin. This standardized microbiota was prepared using fecal donations from a group of 4 healthy volunteers (3 females, 1 male, age 38.8 ± 3.9 years; BMI (body mass index) 24.2 ± 1.5 kg/m²). Individuals provided signed informed consent before participation, were non-smokers and had not used antibiotics, prebiotics, probiotics or laxatives within 1 month before the stool donation.

At the start of the adaptation period the microbiota was allowed to adapt to the model conditions and SIEM for 16 h. After the adaptation period the 120 h test period started in which lactulose was added to TIM-2 once daily.

### Addition of Test Product:

Lactulose was added to the system at daily doses of 2 g, 3 g, 4 g, and 5 g lactulose. Each dose as well as the control experiment was studied in duplicate (n = 2). The test period of the TIM-2 experiments lasted 120 h (5 consecutive days).

### Sampling from TIM-2:

Metabolites like the short-chain fatty acids (SCFA) and ammonia produced in TIM-2 were continuously removed from the lumen by a semipermeable membrane unit. This dialysate was collected at the start of the test period and after 24, 48, 72, 96, and 120 h.

At the beginning and end of the experiment (t = 0 h and t = 120 h) luminal samples were used to investigate the composition of the microbiota. The samples were snap frozen in liquid nitrogen and stored at ≤ -72 °C until analysis.

### Sodium Hydroxide Usage (pH):

The pH was kept at a value of 5.8 by automatic titration with 2 M NaOH, the consumption of NaOH was monitored.

### Short-Chain Fatty Acids (SCFA):

The lumen and dialysate fractions of TIM-2 were analyzed using gas chromatography for SCFA (acetate, propionate and butyrate). Dialysate samples were directly used. Lumen samples were centrifuged (12,000 rpm at 4 °C for 10 min).

### Ammonia:

Samples for ammonia analysis were centrifuged as described above. Ammonia was determined based on the Berthelot reaction.

### 16S rDNA Amplicon Sequencing:

The bacterial population in the TIM-2 samples was analyzed using Next Generation sequencing. Total DNA from the collected TIM-2 lumen samples at the start (t = 0 h) and at the end (t = 120 h) of the experiments was isolated. To determine the recovery of bacterial DNA from the samples, a quantitative polymerase chain reaction (qPCR) using primers specific for the bacterial 16S rRNA gene was used. Changes in the microbiota composition were analyzed by using mass V4 16S rDNA amplicon sequencing. Processing of the sequencing data was done using the Mothur pipeline.

### 10.2 Results and Discussion

### Sodium Hydroxide Usage:

The addition of lactulose in different doses in the test period showed a dose dependent increased use of NaOH during the TIM-2 experiments as shown in Table 1.

**Table 1: Cumulative sodium hydroxide consumption in mL during TIM-2 runs (mean of n = 2)**

| | Control | Lactulose 2 g | Lactulose 3 g | Lactulose 4 g | Lactulose 5 g |
|---|---|---|---|---|---|
| 0 h | 19 | 19 | 20 | 20 | 18 |
| 24 h | 48 | 58 | 65 | 72 | 69 |
| 48 h | 71 | 89 | 105 | 113 | 117 |
| 72 h | 90 | 115 | 136 | 151 | 157 |
| 96 h | 107 | 139 | 169 | 185 | 193 |
| 120 h | 125 | 162 | 202 | 223 | 223 |

### Markers of Saccharolytic Fermentation - SCFA Production:

Table 2a shows the cumulative total SCFA (acetate, propionate and butyrate) production during the 120 h test period in TIM-2. The different lactulose doses all show a higher SCFA production as compared to the control. Table 2b shows the cumulative butyrate production during the 120 h test period in TIM-2.

**Table 2a: Cumulative production of total short chain fatty acids (SCFA) in mmol in TIM-2 runs (mean of n = 2). Values at the start of the test period were set to zero.**

| | Control | Lactulose 2 g | Lactulose 3 g | Lactulose 4 g | Lactulose 5 g |
|---|---|---|---|---|---|
| 0 h | 0 | 0 | 0 | 0 | 0 |
| 24 h | 62 | 85 | 85 | 97 | 84 |
| 48 h | 129 | 163 | 175 | 216 | 214 |
| 72 h | 194 | 234 | 256 | 310 | 309 |
| 96 h | 257 | 318 | 340 | 405 | 430 |
| 120 h | 320 | 403 | 427 | 500 | 520 |

**Table 2b: Cumulative production of butyrate in mmol in TIM-2 runs (mean of n = 2). Values at the start of the test period were set to zero.**

| | Control | Lactulose 2 g | Lactulose 3 g | Lactulose 4 g | Lactulose 5 g |
|---|---|---|---|---|---|
| 0 h | 0 | 0 | 0 | 0 | 0 |
| 24 h | 7 | 14 | 12 | 12 | 8 |
| 48 h | 18 | 27 | 27 | 30 | 29 |
| 72 h | 31 | 39 | 40 | 47 | 53 |
| 96 h | 44 | 52 | 54 | 70 | 84 |
| 120 h | 58 | 69 | 68 | 93 | 118 |

Lactulose increased the levels of SCFA and butyrate. Butyrate is used preferentially as an energy source by the gut mucosa. Furthermore, butyrate has an anti-inflammatory effect with regard to cytokines and inhibits production of reactive oxygen species.

### Markers of Proteolytic Fermentation - Ammonia Production:

The total amount of ammonia was measured and is shown in Table 3. With increasing doses of lactulose there was a decreased mean ammonia production.

**Table 3: Cumulative ammonia production in mmol during the 120 h test period in TIM-2 runs (mean of n = 2)**

| | Control | Lactulose 2 g | Lactulose 3 g | Lactulose 4 g | Lactulose 5 g |
|---|---|---|---|---|---|
| 0 h | 0 | 0 | 0 | 0 | 0 |
| 24 h | 10 | 9 | 9 | 6 | 5 |
| 48 h | 31 | 29 | 26 | 15 | 12 |
| 72 h | 55 | 48 | 45 | 25 | 22 |
| 96 h | 82 | 68 | 64 | 38 | 32 |
| 120 h | 108 | 88 | 85 | 51 | 41 |

Lactulose decreased the levels of ammonia.

### Microbiota Composition:

Analysis with mass V4 16S rDNA amplicon sequencing resulted in an overview of the relative abundance of the bacterial genera Bifidobacterium and Lactobacillus in the microbiota lumen samples collected from the TIM-2 experiments after 120 h exposure to the different test conditions. The genus level abundances for the control and test conditions after 120 h in TIM-2 are shown in Table 4. Lactulose increased the abundance of Bifidobacterium and Lactobacillus, indicative of a prebiotic effect.

**Table 4: Average number of the bacterial genera Bifidobacterium and Lactobacillus after 120 h of exposure in TIM-2**

| | Control | Lactulose 2 g | Lactulose 3 g | Lactulose 4 g | Lactulose 5 g |
|---|---|---|---|---|---|
| Bifidobacterium | 28,345 | 24,781 | 17,424 | 135,876 | 116,792 |
| Lactobacillus | 82 | 1,623 | 1,504 | 12,047 | 912 |

## Claims

1. Composition comprising
a) 60-99 wt% of lactulose, based on the total weight of the composition;
b) at least one vitamin selected from vitamin A, vitamin C, vitamin D and vitamin E; and
c) at least one member of the group consisting of calcium, phosphorus, zinc, selenium, and choline,
wherein the composition is in the form of a solid, and,
wherein at least 88 wt% of the lactulose has a particle size in the range of from 100 to 400µm.

2. Composition according to claim 1, wherein the total weight of the composition is 1.9-6.5 g, preferably 2.0-6.0 g, more preferably 2.1-5.2 g.

3. Composition according to claim 1 or 2, wherein the total weight of the composition is 1.9-3.9 g, preferably 2.0-3.7 g, more preferably 2.1-3.5 g.

4. Composition according to any one of claims 1 to 3 which comprises
b1) 90-220 µg RE, preferably 100-200 µg RE, more preferably 115-188 µg RE vitamin A per g of the composition;
b2) 10-55 mg, preferably 15-45 mg, more preferably 20-38 mg vitamin C per g of the composition; and
c4) 10-40 µg, preferably 12-30 µg, more preferably 15-26 µg selenium per g of the composition.

5. Composition according to claim 4, wherein the total weight of the composition is 1.9-6.5 g, preferably 2.0-6.0 g, more preferably 2.1-5.2 g, particularly preferably 3.0-3.5 g.

6. Composition according to any one of claims 1 to 3 which comprises
b3) 1.1-2.5 µg, preferably 1.4-2.1 µg, more preferably 1.6-1.8 µg vitamin D per g of the composition;
c1) 40-70 mg, preferably 48-62 mg, more preferably 52-56 mg calcium per g of the composition;
c2) 25-60 mg, preferably 35-50 mg, more preferably 40-43 mg phosphorus per g of the composition; and
c3) 2.5-4.8 mg, preferably 2.8-4.0 mg, more preferably 3.2-3.5 mg zinc per g of the composition.

7. Composition according to claim 6, wherein the total weight of the composition is 2.6-3.5 g, preferably 2.8-3.3 g, more preferably 2.9-3.1 g.

8. Composition according to any one of claims 1 to 3 which comprises
b4) 1.0-2.6 mg, preferably 1.1-2.2 mg, more preferably 1.2-1.9 mg alpha-TE vitamin E per g of the composition; and
c5) 12-118 mg, preferably 15-30 mg, more preferably 17-26 mg choline per g of the composition.

9. Composition according to claim 8, wherein the total weight of the composition is 3.0-6.5 g, preferably 3.2-6.0 g, more preferably 3.3-5.2 g, particularly preferably 4.1-4.8 g.

10. Dose unit comprising a composition as defined in any one of claims 1 to 9.

11. Dose unit according to claim 10 comprising a composition as defined in any one of claims 1, 4, 6 or 8 providing 1.9-6.5 g, preferably 2.0-6.0 g, more preferably 2.1-5.2 g of the composition as defined in any one of claims 1, 4, 6 or 8.

12. Dose unit according to claim 10 or 11 comprising a composition as defined in any one of claims 1, 4, 6 or 8 providing 1.9-3.9 g, preferably 2.0-3.7 g, more preferably 2.1-3.5 g of the composition as defined in any one of claims 1, 4, 6 or 8.

13. Dose unit according to claim 10 comprising a composition as defined in any one of claims 1, 4, 6 or 8 providing 1.8-5.5 g, preferably 1.9-5.2 g, more preferably 2.0-4.5 g of lactulose.

14. Dose unit according to claim 10 or 11 comprising a composition as defined in any one of claims 1, 4, 6 or 8 providing 1.8-3.6 g, preferably 1.9-3.4 g, more preferably 2.0-3.2 g of lactulose.

15. Composition as defined in any one of claims 1 to 9 for use in maintaining or improving gut health in humans.

## Patentansprüche

1. Zusammensetzung, umfassend
a) 60-99 Gew.-% Lactulose, bezogen auf das Gesamtgewicht der Zusammensetzung;
b) mindestens ein Vitamin, ausgewählt aus Vitamin A, Vitamin C, Vitamin D und Vitamin E; und
c) mindestens ein Element der Gruppe bestehend aus Calcium, Phosphor, Zink, Selen und Cholin,
wobei die Zusammensetzung in Form eines Feststoffs vorliegt und,
wobei mindestens 88 Gew.-% der Lactulose eine Partikelgröße im Bereich von 100 bis 400 µm aufweist.

2. Zusammensetzung nach Anspruch 1, wobei das Gesamtgewicht der Zusammensetzung 1,9-6,5 g, bevorzugt 2,0-6,0 g, und besonders bevorzugt 2,1-5,2 g beträgt.

3. Zusammensetzung nach Anspruch 1 oder 2, wobei das Gesamtgewicht der Zusammensetzung 1,9-3,9 g, bevorzugt 2,0-3,7 g, und besonders bevorzugt 2,1-3,5 g beträgt.

4. Zusammensetzung nach einem der Ansprüche 1 bis 3, die Folgendes umfasst
b1) 90-220 µg RE, bevorzugt 100-200 µg RE, und besonders bevorzugt 115-188 µg RE Vitamin A pro g der Zusammensetzung;
b2) 10-55 mg, bevorzugt 15-45 mg, und besonders bevorzugt 20-38 mg Vitamin C pro g der Zusammensetzung; und
c4) 10-40 µg, bevorzugt 12-30 µg, und besonders bevorzugt 15-26 µg Selen pro g der Zusammensetzung.

5. Zusammensetzung nach Anspruch 4, wobei das Gesamtgewicht der Zusammensetzung 1,9-6,5 g, bevorzugt 2,0-6,0 g, und besonders bevorzugt 2,1-5,2 g, besonders bevorzugt 3,0-3,5 g beträgt.

6. Zusammensetzung nach einem der Ansprüche 1 bis 3, die Folgendes umfasst
b3) 1,1-2,5 µg, bevorzugt 1,4-2,1 µg, und besonders bevorzugt 1,6-1,8 µg Vitamin D pro g der Zusammensetzung;
c1) 40-70 mg, bevorzugt 48-62 mg, und besonders bevorzugt 52-56 mg Calcium pro g der Zusammensetzung;
c2) 25-60 mg, bevorzugt 35-50 mg, und besonders bevorzugt 40-43 mg Phosphor pro g der Zusammensetzung; und
c3) 2,5-4,8 mg, bevorzugt 2,8-4,0 mg, und besonders bevorzugt 3,2-3,5 mg Zink pro g der Zusammensetzung.

7. Zusammensetzung nach Anspruch 6, wobei das Gesamtgewicht der Zusammensetzung 2,6-3,5 g, bevorzugt 2,8-3,3 g, und besonders bevorzugt 2,9-3,1 g beträgt.

8. Zusammensetzung nach einem der Ansprüche 1 bis 3, die Folgendes umfasst
b4) 1,0-2,6 mg, bevorzugt 1,1-2,2 mg, und besonders bevorzugt 1,2-1,9 mg Alpha-TE Vitamin E pro g der Zusammensetzung; und
c5) 12-118 mg, bevorzugt 15-30 mg, und besonders bevorzugt 17-26 mg Cholin pro g der Zusammensetzung.

9. Zusammensetzung nach Anspruch 8, wobei das Gesamtgewicht der Zusammensetzung 3,0-6,5 g, bevorzugt 3,2-6,0 g, und besonders bevorzugt 3,3-5,2 g, besonders bevorzugt 4,1-4,8 g beträgt.

10. Dosiseinheit, die eine Zusammensetzung nach einem der Ansprüche 1 bis 9 umfasst.

11. Dosiseinheit nach Anspruch 10, umfassend eine Zusammensetzung nach einem der Ansprüche 1, 4, 6 oder 8, die 1,9-6,5 g, bevorzugt 2,0-6,0 g, und besonders bevorzugt 2,1-5,2 g der Zusammensetzung nach einem der Ansprüche 1, 4, 6 oder 8 bereitstellt.

12. Dosiseinheit nach Anspruch 10 oder 11, umfassend eine Zusammensetzung nach einem der Ansprüche 1, 4, 6 oder 8, die 1,9-3,9 g, bevorzugt 2,0-3,7 g, und besonders bevorzugt 2,1-3,5 g der Zusammensetzung nach einem der Ansprüche 1, 4, 6 oder 8 bereitstellt.

13. Dosiseinheit nach Anspruch 10, umfassend eine Zusammensetzung nach einem der Ansprüche 1, 4, 6 oder 8, die 1,8-5,5 g, bevorzugt 1,9-5,2 g, und besonders bevorzugt 2,0-4,5 g Lactulose bereitstellt.

14. Dosiseinheit nach Anspruch 10 oder 11, umfassend eine Zusammensetzung nach einem der Ansprüche 1, 4, 6 oder 8, die 1,8-3,6 g, bevorzugt 1,9-3,4 g, und besonders bevorzugt 2,0-3,2 g Lactulose bereitstellt.

15. Zusammensetzung nach einem der Ansprüche 1 bis 9 zur Verwendung bei der Erhaltung oder Verbesserung der Darmgesundheit beim Menschen.

## Revendications

1. Composition comprenant
a) 60 à 99 % en poids de lactulose, par rapport au poids total de la composition ;
b) au moins une vitamine choisie parmi la vitamine A, la vitamine C, la vitamine D et la vitamine E ; et
c) au moins un élément du groupe constitué par le calcium, le phosphore, le zinc, le sélénium et la choline,
la composition étant sous la forme d'un solide et
au moins 88 % en poids du lactulose ayant une taille des particules dans la plage allant de 100 et 400 µm.

2. Composition selon la revendication 1, le poids total de la composition étant de 1,9 à 6,5 g, de préférence de 2,0 à 6,0 g, plus préférablement de 2,1 à 5,2 g.

3. Composition selon la revendication 1 ou 2, le poids total de la composition étant de 1,9 à 3,9 g, de préférence de 2,0 à 3,7 g, plus préférablement de 2,1 à 3,5 g.

4. Composition selon l'une quelconque des revendications 1 à 3 qui comprend
b1) 90 à 220 µg d'ER, de préférence 100 à 200 µg d'ER, plus préférablement 115 à 188 µg d'ER de vitamine A par g de la composition ;
b2) 10 à 55 mg, de préférence 15 à 45 mg, plus préférablement 20 à 38 mg de vitamine C par g de la composition ; et
c4) 10 à 40 µg, de préférence 12 à 30 µg, plus préférablement 15 à 26 µg de sélénium par g de la composition.

5. Composition selon la revendication 4, le poids total de la composition étant de 1,9 à 6,5 g, de préférence de 2,0 à 6,0 g, plus préférablement de 2,1 à 5,2 g, particulièrement préférablement de 3,0 à 3,5 g.

6. Composition selon l'une quelconque des revendications 1 à 3 qui comprend
b3) 1,1 à 2,5 µg, de préférence 1,4 à 2,1 µg, plus préférablement 1,6 à 1,8 µg de vitamine D par g de la composition ;
c1) 40 à 70 mg, de préférence 48 à 62 mg, plus préférablement 52 à 56 mg de calcium par g de la composition ;
c2) 25 à 60 mg, de préférence 35 à 50 mg, plus préférablement 40 à 43 mg de phosphore par g de la composition ; et
c3) 2,5 à 4,8 mg, de préférence 2,8 à 4,0 mg, plus préférablement 3,2 à 3,5 mg de zinc par g de la composition.

7. Composition selon la revendication 6, le poids total de la composition étant de 2,6 à 3,5 g, de préférence de 2,8 à 3,3 g, plus préférablement de 2,9 à 3,1 g.

8. Composition selon l'une quelconque des revendications 1 à 3 qui comprend
b4) 1,0 à 2,6 mg, de préférence 1,1 à 2,2 mg, plus préférablement 1,2 à 1,9 mg d'E-alpha-T de vitamine E par g de la composition ; et
c5) 12 à 118 mg, de préférence 15 à 30 mg, plus préférablement 17 à 26 mg de choline par g de la composition.

9. Composition selon la revendication 8, le poids total de la composition étant de 3,0 à 6,5 g, de préférence de 3,2 à 6,0 g, plus préférablement de 3,3 à 5,2 g, particulièrement préférablement de 4,1 à 4,8 g.

10. Dose unitaire comprenant une composition telle que définie dans l'une quelconque des revendications 1 à 9.

11. Dose unitaire selon la revendication 10 comprenant une composition telle que définie dans l'une quelconque des revendications 1, 4, 6 ou 8 fournissant 1,9 à 6,5 g, de préférence 2,0 à 6,0 g, plus préférablement 2,1 à 5,2 g de la composition telle que définie dans l'une quelconque des revendications 1, 4, 6 ou 8.

12. Dose unitaire selon la revendication 10 ou 11 comprenant une composition telle que définie dans l'une quelconque des revendications 1, 4, 6 ou 8 fournissant 1,9 à 3,9 g, de préférence 2,0 à 3,7 g, plus préférablement 2,1 à 3,5 g de la composition telle que définie dans l'une quelconque des revendications 1, 4, 6 ou 8.

13. Dose unitaire selon la revendication 10 comprenant une composition telle que définie dans l'une quelconque des revendications 1, 4, 6 ou 8 fournissant 1,8 à 5,5 g, de préférence 1,9 à 5,2 g, plus préférablement 2,0 à 4,5 g de lactulose.

14. Dose unitaire selon la revendication 10 ou 11 comprenant une composition telle que définie dans l'une quelconque des revendications 1, 4, 6 ou 8 fournissant 1,8 à 3,6 g, de préférence 1,9 à 3,4 g, plus préférablement 2,0 à 3,2 g de lactulose.

15. Composition telle que définie dans l'une quelconque des revendications 1 à 9 destinée à être utilisée en maintien ou amélioration de la santé intestinale chez l'homme.
